(19) Europäisches Patentamt
European Patent Office
Office européen des brevets

(11) Veröffentlichungsnummer: **0 346 760**
**A2**

(12) **EUROPÄISCHE PATENTANMELDUNG**

(21) Anmeldenummer: **89110350.9**

(22) Anmeldetag: **08.06.89**

(51) Int. Cl.4: **C07D 493/10 , A61K 31/365 , //(C07D493/10,311:00,307:00)**

(30) Priorität: **14.06.88 DE 3820179**
**31.08.88 DE 3829452**

(43) Veröffentlichungstag der Anmeldung:
**20.12.89 Patentblatt 89/51**

(84) Benannte Vertragsstaaten:
**BE CH DE FR GB IT LI NL**

(71) Anmelder: **HOECHST AKTIENGESELLSCHAFT**
**Postfach 80 03 20**
**D-6230 Frankfurt am Main 80(DE)**

(72) Erfinder: **Hammann, Peter, Dr.**
**Mörikestrasse 6**
**D-6233 Kelkheim(DE)**
Erfinder: **Wink, Joachim, Dr.**
**Bieberer Strasse 133**
**D-6050 Offenbach am Main(DE)**
Erfinder: **Seibert, Gerhard, Prof.Dr.**
**Gläserweg 21**
**D-6100 Darmstadt(DE)**
Erfinder: **Winkler, Irvin, Dr.**
**In den Eichen 40**
**D-6237 Liederbach(DE)**

(54) **Nigericinderivate, Verfahren zu deren Herstellung, diese enthaltende Mittel und Verwendung derselben.**

(57) Die Erfindung betrifft Nigericinderivate der Formel I

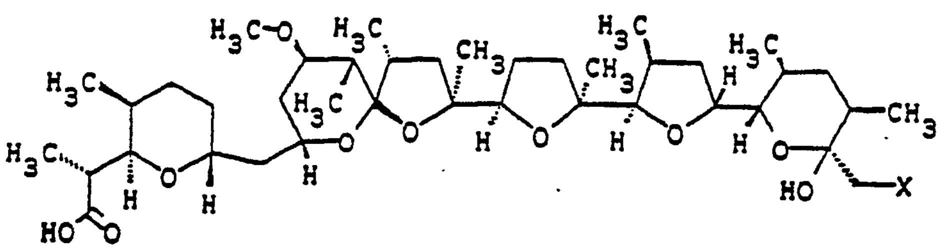

(I)

in der X die angegebenen Bedeutungen hat. Die erfindungsgemäßen Nigericinderivate zeigen antivirale und antibakterielle Wirksamkeit.

EP 0 346 760 A2

## Nigericinderivate, Verfahren zu deren Herstellung, diese enthaltende Mittel und Verwendung derselben

Der Polyether Nigericin, der durch Kultivierung von Streptomyces hygroscopicus erhalten werden kann, ist u. a. beschrieben in J. Berger et al., Am. Chem. Soc. 73, 5295 (1951)

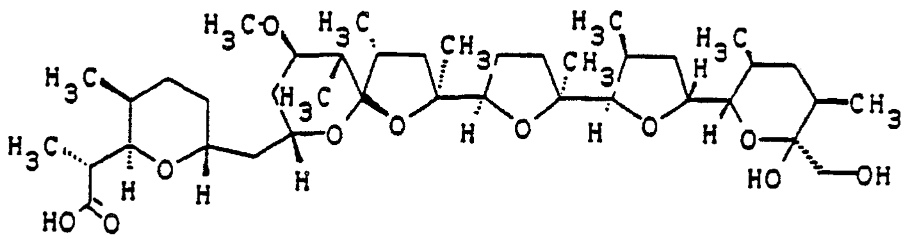

und wurde bislang als Antibiotikum eingesetzt.

Einige Derivate des Nigericins sind ebenfalls bekannt (JP 72 01,288 und T. Kubota, J. Chem. Soc. (C) 1970, 695, US 3,995,027 und US 3,832,358), auch sie wurden bislang als Antibiotika oder gegen Tierviren eingesetzt.

Eine antivirale Wirksamkeit von Nigericin oder Nigericinderivaten, insbesondere gegen Viren, die bei Menschen Erkrankungen hervorrufen können, ist bisher noch nicht bekannt geworden. Lediglich in den älteren deutschen Anmeldungen P 38 00 598 und P 38 11 016 ist eine solche antivirale Wirksamkeit vorgeschlagen worden; diese ist jedoch in vielerlei Hinsicht noch nicht befriedigend.

Es wurde nun gefunden, daß bestimmte Nigericinderivate hochwirksame antivirale und antibakterielle Mittel sind.

Die Erfindung betrifft nun Nigericinderivate der Formel I

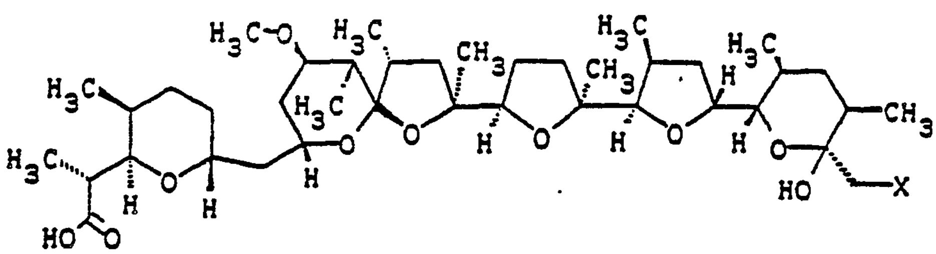

(I)

in der

X F, Cl, Br, J, SCN, CN, $NO_2$ oder $N_3$ bedeutet oder einen Substituenten der Formel II darstellt

$Y - R^1$ (II)

worin

$R^1$ Wasserstoff, $C_1$-$C_{10}$-Alkyl, $C_2$-$C_{10}$Alkenyl, $C_3$-$C_8$-Cycloalkyl, Trimethylsilyl, Triethylsilyl, Dimethylphenylsilyl, Dimethylcyclohexylsilyl, Dimethyltertiärbutylsilyl, Diphenyltertiärbutylsilyl, Pyrrolyl, Benzpyrrolyl, Imidazolyl, Benzimidazolyl, Triazolyl oder Tetrazolyl oder Phenyl bedeutet, wobei die genannten Aryl-, Heteroaryl- oder benzokondensierten Heteroarylreste gegebenenfalls mit $C_1$-$C_4$-Alkyl, $C_1$-$C_4$-Alkoxy, $C_1$-$C_4$-Alkoxycarbonyl, Carboxyl, F, Cl, Br, J, $NO_2$ oder CN mono- oder disubstituiert sind und worin

Y O oder S bedeutet, wobei für

$OR^1$ die Bedeutungen Wasserstoff, Pyrrol, Benzpyrrol, Imidazol, Benzimidazol, Triazol und Tetrazol für $R^1$ ausgenommen sind und für

$SR^1$ die Bedeutungen Trimethylsilyl, Triethylsilyl, Dimethylphenylsilyl, Dimethylcyclohexylsilyl, Diphenyltertiärbutylsilyl und Dimethyltertiärbutylsilyl für $R^1$ ausgenommen sind oder worin

X einen Rest der Formel III

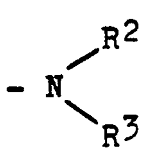

(III)

darstellt,
worin
$R_2$ und $R_3$ gleich oder verschieden sind und Wasserstoff, $C_1$-$C_{18}$-Alkyl, $C_2$-$C_{18}$-Alkenyl, $C_3$-$C_8$-Cycloalkyl oder Phenyl bedeuten, wobei die genannten Alkyl-, Alkenyl- und Cycloalkyl-Reste ihrerseits mit Phenyl, Naphthyl oder Thienyl monosubstituiert und die genannten Phenyl- und Alkylreste darüber hinaus noch mit $COOR^4$ monosubstituiert sein können, wobei $R^4$ Wasserstoff oder $C_1$-$C_6$-Alkyl bedeutet
oder worin
$R^2$ und $R^3$ zusammen mit dem N-Atom, an dem sie gebunden sind, einen 5- oder 6-gliedrigen N-haltigen Alkylring bilden, wobei eine $CH_2$-Einheit des Rings, welche nicht direkt benachbart zum N-Atom steht, ihrerseits durch NH, O oder S ersetzt sein kann,
sowie deren physiologisch verträgliche Salze.

Insbesondere betrifft die Erfindung Nigericinderivate der Formel I wie oben definiert, wobei jedoch mindestens eine der nachfolgenden Bedingungen erfüllt ist:
$R^1$ bedeutet Wasserstoff, $C_1$-$C_8$-Alkyl, $C_3$-$C_8$-Alkenyl, $C_3$-$C_8$-Cycloalkyl, Trimethylsilyl, Triethylsilyl, Dimethyltertiärbutylsilyl, Dimethylphenylsilyl, Dimethylcyclohexylsilyl, Dimethyltertiärbutylsilyl, Pyrrolyl, Benzpyrrolyl, Tetrazolyl, oder Phenyl, wobei die genannten Aryl-, Heteroaryl- oder benzokondensierten Heteroarylreste gegebenenfalls mit $C_1$-$C_4$-Alkyl, $C_1$-$C_4$-Alkoxy, $C_1$-$C_4$-Alkoxycarbonyl, Carboxyl, F, Cl, Br, J, $NO_2$ oder CN monosubstituiert sind;
$R^2$ und $R^3$ sind gleich oder verschieden und bedeuten Wasserstoff, $C_1$-$C_8$-Alkyl, $C_5$-$C_6$-Cycloalkyl oder Phenyl, wobei die genannten Alkyl- und Cycloalkylreste ihrerseits mit Phenyl monosubstituiert und die Phenyl-und Alkylreste darüber hinaus noch mit $COOR^4$ monosubstituiert sein können, wobei $R^4$ Wasserstoff oder $C_1$-$C_4$-Alkyl bedeutet.

Ganz besonders bevorzugt sind Nigericinderivate der Formel I wie oben definiert, wobei jedoch mindestens eine der nachfolgenden Bedingungen erfüllt ist:
X bedeutet F, Cl, Br, SCN, CN, $NO_2$ oder $N_3$ oder einen Substituenten der Formel II, worin
$R^1$ Wasserstoff, $C_1$-$C_6$-Alkyl, $C_5$-$C_6$-Cycloalkyl, Trimethylsilyl, Triethylsilyl, Diphenyltertiärbutylsilyl, Dimethylphenylsilyl, Dimethylcyclohexylsilyl, Dimethyltertiärbutylsilyl, Pyrrolyl, Benzpyrrolyl, Tetrazolyl, Methyltetrazolyl oder Phenyl bedeutet;
$R^2$ und $R^3$ sind gleich oder verschieden und bedeuten Wasserstoff, $C_1$-$C_4$-Alkyl oder Phenyl, wobei die Alkylreste ihrerseits mit Phenyl monosubstituiert sein können;
$R^2$ und $R^3$ bilden zusammen mit dem N-Atom, an dem sie gebunden sind, einen Morpholin-, Thiomorpholin- oder Piperazinring.

Des weiteren betrifft die Erfindung ein Verfahren zur Herstellung von Nigericinderivaten der Formel I, wie oben definiert, dadurch gekennzeichnet, daß man Nigericin (Formel I: X = OH) mit einer einen nukleofugen Rest liefernden Verbindung IV
Hal - Z (IV)
worin
Hal Chlor, Brom oder Jod und

Z $-SO_2CH_3$, $-SO_2CF_3$, 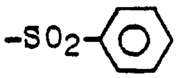 oder 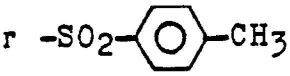

bedeuten,
zu einer Verbindung der Formel V

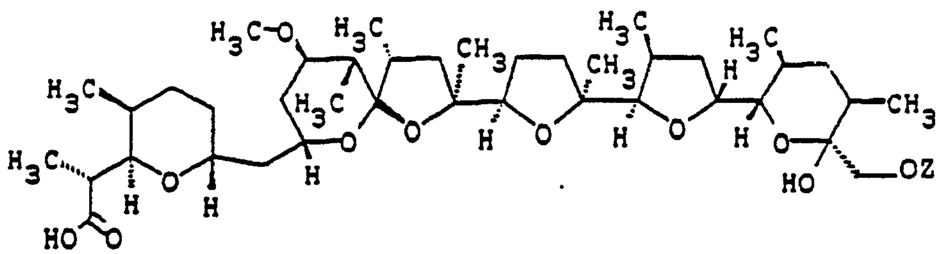
(V)

wobei Z die oben zu Formel IV angegebenen Bedeutungen hat, umsetzt,
und anschließend

a) die Verbindung V mit $NaN_3$ oder einer Verbindung der Formel VI umsetzt,

$M - X'$ (VI)

worin

M Li, Na, K oder ein quartäres, organisches Ammoniumfragment bedeutet und

X′ F, Cl, Br, J, SCN, CN oder $NO_2$ bedeutet, wobei man eine Verbindung der Formel I′ erhält,

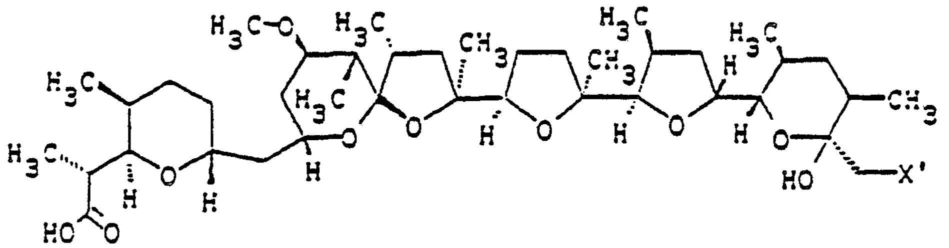 (I')

worin

X′ $N_3$ ist oder die oben zu Formel VI angegebenen Bedeutungen hat

oder daß man

b) die Verbindung der Formel V mit einem Alkohol, Thiol oder Amin der Formel VII bzw. VII′ umsetzt,

$HY - R^1$ (VII) 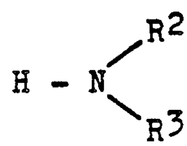 (VII')

worin

Y, $R^2$ und $R^3$ die oben zu Formel I angegebenen Bedeutungen haben und

$R^1$ bis auf Trimethylsilyl, Triethylsilyl, Dimethylphenylsilyl, Dimethylcyclohexylsilyl, Diphenyltertiärbutylsilyl und Dimethyltertiärbutylsilyl

die oben zu Formel I angegebenen Bedeutungen hat,

wobei man eine Verbindung der Formel I erhält, in der

X $Y-R^1$ oder 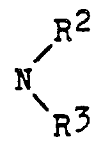

bedeutet, wobei Y, $R^2$ und $R^3$

die oben zu Formel I angegebenen Bedeutungen haben und $R^1$ bis auf Trimethylsilyl, Triethylsilyl, Dimethylphenylsilyl, Dimethylcyclohexylsilyl, Diphenyltertiärbutylsilyl und Dimethyltertiärbutylsilyl die oben zu Formel I angegebenen Bedeutungen hat

oder daß man

c) Nigericin umsetzt mit einer Verbindung der Formel VIII

Hal′ - Sil (VIII)

worin

Hal′ Chlor oder Brom und

Sil Trimethylsilyl, Triethylsilyl, Dimethylphenylsilyl, Dimethylcyclohexylsilyl, Diphenyltertiärbutylsilyl oder Dimethyltertiärbutylsilyl bedeutet,

wobei man eine Verbindung der Formel I erhält, in der

X $Y-R^1$ bedeutet, wobei Y Sauerstoff ist und

$R^1$ Trimethylsilyl, Triethylsilyl, Dimethylphenylsilyl, Dimethylcyclohexylsilyl, Diphenyltertiärbutylsilyl oder Dimethyltertiärbutylsilyl bedeutet,

und daß man gegebenenfalls anschließend die Verbindungen der Formel I in ihre physiologisch verträglichen Salze überführt.

Ebenso betrifft die Erfindung die Anwendung der vorgenannten Verbindung als Arzneimittel; insbesondere als antibakteriell und antiviral wirksames Arzneimittel.

Alle genannten Alkyl- oder Alkenylreste mit drei und mehr C-Atomen können sowohl geradkettig als auch verzweigt sein. Unter Aryl und Heteroarylresten werden aromatische oder heteroaromatische Kohlenwasserstoffe verstanden, wie Phenyl, Naphthyl, Pyrrolyl, Imidazolyl, Tetrazolyl oder Thienyl und unter benzokondensierten Heteroarylresten werden benzoannelierte heteroaromatische Kohlenwasserstoffe verstanden, wie Benzpyrrolyl oder Benzimidazol.

Bei zwei- oder mehrfach substituierten Aryl-, Heteroaryl-oder benzokondensierten Heteroarylresten können die Substituenten sowohl gleich als auch verschieden sein.

Bei den für $R^1$ genannten Pyrrolyl- Benzpyrrolyl-, Imidazolyl-, Benzimidazolyl-, Triazolyl- und Tetrazolyl-Resten handelt es sich bevorzugt um Pyrrol-2-yl, Pyrrol-3-yl, Benzpyrrol-2-yl, Benzpyrrol-3-yl, Imidazol-2-yl, Imididazol-4-yl, Imidazol-5-yl, Benzimidazol-2-yl, 1,2,4-Triazol-3-yl, 1,2,4-Triazol-5-yl oder 1,2,3,4-Tetrazol-5-yl.

Unter einem quartären organischen Ammoniumfragment werden die kationischen quartären Reste von Ammoniumsalzen verstanden, wie Tetraphenylammonium oder Tetrabutylammonium. Als geeignete Salze seien beispielsweise genannt: Tetrabutylammoniumchlorid, Tetrabutylammoniumbromid oder Tetraphenylammoniumcyanid.

Unter einem nukleofugen Rest werden solche Substituenten verstanden, die bei einer nukleophilen Substitution leicht vom betreffenden Molekül abgespalten werden können und somit den Eintritt eines nukleophilen Agens erleichtern bzw. erst möglich machen. Gängige nukleofuge Gruppen sind beispielsweise Cl, Br, J, Methylsulfonate, Phenylsulfonate oder Tosylsulfonate.

Im folgenden werden die Verfahren a) bis c), die es ermöglichen, die unterschiedlich substituierten Nigericinderivate herzustellen, näher beschrieben.

Mit Hilfe der Verfahrensvarianten a) und b) lassen sich sämtliche Substituenten, bis auf die Silylverbindungen, beispielsweise durch nukleophile Substitution des Sulfonsäurederivats in das Molekül einführen. Die Einführung der Silylsubstituenten gemäß Verfahrensvariante c) gelingt durch einfache Umsetzung von Nigericin (Formel I: X = OH) mit den entsprechenden Silylhalogeniden, bevorzugt -chloriden.

Zur Herstellung der Sulfonsäurederivate geht man am besten so vor, daß man Nigericin (Formel I: X = OH) in äquimolaren Mengen oder in einem bis zu 50-fachen Überschuß mit einem Sulfonsäurehalogenid, bevorzugt -chlorid, besonders bevorzugt Methylsulfonylchlorid, umsetzt. Gegebenenfalls kann diese Umsetzung unter Basenzusatz erfolgen. Als Basen kommen beispielsweise Triethylamin, Pyridin oder Lutidin in Frage. Eine Variante des Verfahrens besteht darin, daß man in einem geeigneten, vorzugsweise inerten Lösungsmittel wie Chloroform, Methylenchlorid, Tetrahydrofuran (THF), Essigester oder Dioxan arbeitet. Auch hier kann der Überschuß an Sulfonsäurehalogenid bis zur 50-fachen Menge betragen.

Die Reaktionstemperaturen liegen dabei zwischesn -70° C und +100° C, vorzugsweise bei Verwendung eines Lösungsmittels zwischen dem Festpunkt und dem Siedepunkt des Lösungsmittels, insbesondere zwischen -70° C und +40° C.

Die Reaktionszeiten betragen 1 bis 180 Stunden; bevorzugt 1 bis 48 Stunden, besonders bevorzugt 1 bis 8 Stunden. Die Beendigung der Reaktion kann beispielsweise mittels dünnschichtchromatographischer Kontrolle (DC-Kontrolle) bestimmt werden.

Die als Ausgangssubstanzen verwendeten Sulfonsäurehalogenide sind käuflich.

Nigericin kann nach dem von J. Berger (s. loc. cit. Am. Chem. Soc.) beschriebenen Verfahren hergestellt werden. Es kann aber auch gemäß dem in der Deutschen Patentanmeldung P 37 00 325.9 vorgeschlagenen Verfahren hergestellt werden, wobei Nigericin neben Amycin bei der Kultivierung von Streptomyces parvulus DSM 3816 entsteht. Das Nigericin kann aus dem Mycel mittels Hexan extrahiert und nach Aufkonzentrierung auskristallisiert werden.

Bei der Verfahrensvarianten a) geht man am besten so vor, daß man das Sulfonsäurederivat der Formel V mit Natriumazid oder mit dem Salz der Formel VI in äquimolaren Mengen oder in einem bis zu 50-fachen Überschuß des Salzes in einem inerten Lösungsmittel wie Dimethylformamid (DMF), Tetrahydrofuran (THF), Aceton, Acetonitril, Toluol, Dimethylsulfoxid (DMSO) oder Chloroform umsetzt. Bevorzugt werden die Lithium-, Natrium- oder Kaliumsalze, besonders bevorzugt die quartären organischen Ammoniumsalze der Formel VI eingesetzt. Bevorzugte Lösungsmittel bei der Umsetzung mit den Ammoniumsalzen sind THF, Chloroform und Toluol. Bei der Umsetzung mit $NaN_3$ ist DMF als Lösungsmittel bevorzugt.

Die Reaktionstemperaturen liegen bei dieser Umsetzung zwischen -70° C und dem Siedepunkt des Lösungsmittels, insbesondere bei -70 bis 100° C, bevorzugt bei 20 bis 90° C, insbesondere bei der Umsetzung mit den quartären Ammoniumsalzen bei 60° C.

Die Reaktionszeiten betragen 10 bis 100 Stunden, bevorzugt 10 bis 60 Stunden. Die Beendigung der Reaktion kann beispielsweise mittels DC-Kontrolle bestimmt werden.

Die als Ausgangsverbindungenen eingesetzten Lithium-, Natrium-, Kalium-, -Fluoride, -Chloride, -Bromide, -Jodide, -Rhodanide, -Cyanide oder -Nitrite sind käuflich; ebenso die quartären organischen Ammoniumsalze wie Tetraphenyl- oder Tetrabenzyl-ammoniumchlorid, -bromid oder -rhodanid.

Die Herstellung der erfindungsgemäßen Verbindungen nach Verfahrensvariante b) gelingt am einfachsten dadurch, daß die beiden Komponenten, das Nigericinsulfonat V und das entsprechende Amin, Thiol oder der Alkohol gemäß Formel VII bzw. VII′ in äquimolaren Mengen oder in bis zu einem 5-fachen Überschuß an VII bzw. VII′ vermengt werden und bei Temperaturen zwischen 50 und 210 °C, bevorzugt bei 60 - 120 °C, bis zur Beendigung der Reaktion erhitzt werden. Die Beendigung der Reaktion läßt sich mittels Dünnschichtchromatographie bestimmen. Die Reaktionszeiten betragen 1 - 200 Stunden, bevorzugt 10 - 60 Stunden.

Eine Variante des Verfahrens b) besteht darin, daß man in einem geeigneten Lösungsmittel, wie Diethylether oder Dimethoxyethan oder THF, Alkoholen, wie Methanol, Ethanol oder Methylglykol, chlorierten Kohlenwasserstoffen, wie Methylenchlorid, Chloroform, Tri- oder Tetrachlorethylen, Toluol, Benzol oder auch polaren Lösungsmitteln wie DMF oder DMSO arbeitet. Sofern die Amine, Thiole oder Alkohole, die umgesetzt werden sollen, flüssig sind, können diese selbstverständlich auch als Lösungsmittel eingesetzt werden. Auch bei der Lösungsmittelvariante kann ein Überschuß von Amin, Thiol oder Alkohol gemäß Formel VII bzw. VII′, der bis zur etwa 5-fachen Menge betragen kann, angewandt werden. Die Reaktionstemperaturen liegen dabei zwischen Raumtemperatur und dem Siedepunkt des Lösungsmittels, wobei Temperaturen im Bereich von 50 bis 130 °C besonders bevorzugt sind.

Eine weitere Variante besteht darin, daß die Umsetzung mit dem Amin, Thiol oder Alkohol, insbesondere die Umsetzung mit dem Alkohol, in Gegenwart einer Base durchgeführt wird, wobei als Basen beispielsweise Triethylamin, DBU (1, 8-Diazabicyclo[5,4,0]undec-7-en) oder Natriumhydrid verwendet werden können.

Die Verbindungen der Formeln VII und VII′ sind käuflich oder nach Standardmethoden der organischen Chemie herstellbar.

Zur Herstellung der Silylderivate des Nigericins gemäß Verfahrensvariante c) geht man am besten so vor, daß man Nigericin (Formel I: X = OH) in äquimolaren Mengen oder in einem bis zu 50-fachen Überschuß mit einem entsprechenden Silylhalogenid, bevorzugt -chlorid, umsetzt. Beispiele für entsprechende Silylhalogenide sind: Trimethylsilylchlorid, Triethylsilylchlorid, Diphenyltertiärbutylsilylchlorid, Dimethylphenylsilylchlorid, Dimethylcyclohexylsilylchlorid oder Dimethyltert.butylsilylchlorid. Gegebenenfalls kann die Umsetzung unter Basenzusatz erfolgen. Als Basen kommen beispielsweise Triethylamin, Pyridin oder Lutidin in Frage. Eine Variante des Verfahrens besteht darin, daß man in einem geeigneten, vorzugsweise inerten Lösungsmittel wie Chloroform, Methylenchlorid, THF, Essigester oder Dioxan arbeitet. Auch hier kann der Überschuß an Silylhalogenid bis zur 50-fachen Menge betragen.

Die Reaktionstemperaturen liegen dabei zwischen -70 °C und +100 °C, vorzugsweise bei Verwendung eines Lösungsmittels zwischen dem Festpunkt und dem Siedepunkt des Lösungsmittels, insbesondere zwischen -70 °C und +40 °C.

Die Herstellung der physiologisch verträglichen Salze erfolgt nach an sich bekannten Methoden durch Umsetzung mit anorganischen oder organischen Säuren oder Basen. Zur Salzbildung sind insbesondere geeignet Chlor-, Brom- der Jodwasserstoffsäure, Phosphorsäure, Schwefelsäure, Methylschwefelsäure, Amidosulfonsäure, Salpetersäure, Ameisensäure, Essigsäure, Propionsäure, Bernsteinsäure, Weinsäure, Milchsäure, Malonsäure, Fumarsäure, Oxalsäure, Zitronensäure, Äpfelsäure, Schleimsäure, Benzoesäure, Salicylsäure, Acetursäure, Naphthalin-1,5-disulfonsäure, Ascorbinsäure, Phenylessigsäure, β-Aminosalicylsäure, Hydroxyethinsulfonsäure, Benzolsulfonsäure oder synthetische Harze, die saure Gruppen enthalten, z. B. solche mit Ionenaustauscherwirkung und Alkali- und Erdalkalihydroxyde, -carbonate oder -bicarbonate sowie physiologisch verträgliche organische Verbindungen, die eine primäre, sekundäre oder tertiäre Aminogruppe tragen.

Die Reinigung, Isolierung und Aufarbeitung der Substanzen erfolgt nach den üblichen Methoden; beispielsweise können die Reaktionsprodukte durch Chromatographie an polaren Trägermaterialien wie Kieselgel oder ®Sephadex LH 20 mit Lösungsmitteln wie niederen Alkanolen wie Methanol oder Chloroform oder Essigester oder Methanol/Chloroform-Mischungen, aber auch extraktive Methoden wie flüssig/flüssig-Extraktion oder fest/flüssig-Extraktion oder durch kristallisation gereinigt werden.

Die erfindungsgemäßen Derivate des Nigericins zeigen ausgezeichnete antivirale Wirkung. Diese antivirale Aktivität wurde in Zellkulturen, die mit Testviren infiziert wurden, getestet. Ebenso zeigen die erfindungsgemäßen Derivate eine antibakterielle Wirkung.

Die erfindungsgemäßen Verbindungen sind aufgrund ihrer pharmakologischen Eigenschaften für die Behandlung von bakteriellen Erkrankungen und Viruserkrankungen, die hervorgerufen werden beispielsweise durch HSV I, II (Herpes simplex I- oder II-Virus) oder auch Picorna- und Retroviren, wie HIV (Human Immunodeficiency Virus), geeignet.

Die Erfindung betrifft daher weiter die Anwendung der erfindungsgemäßen Verbindungen der Formel I sowie deren physiologisch verträgliche Salze bei der Behandlung und Prophylaxe von bakteriellen Erkrankungen oder von Herpes-Virus-, Picorna- und Retroviren-Erkrankungen.

Die neuen Verbindungen können entweder allein oder mit physiologisch verträglichen Hilfs- oder Trägerstoffen vermischt als Arzneimittel angewandt werden. Sie können zu diesem Zweck oral in Dosen von 0,01 - 5,0 mg/kg/Tag, vorzugsweise 0,01 - 1,0 mg/kg/Tag oder parenteral subkutan in Dosen von 0,001 - 2,5 mg/kg/Tag, vorzugsweise 0,001 -1,0 mg/kg/Tag, inbesondere 0,005 - 0,2 mg/kg/Tag, appliziert werden. Besonders bevorzugt ist die topische Anwendung, wobei die Wirkstoffkonzentration in den Salben 0,001 - 1 %, bevorzugt 0,01 - 0,1 %, beträgt. Die Dosierung kann in schweren Fällen auch erhöht werden. In vielen Fällen genügen jedoch auch geringere Dosen.

Die Erfindung umfaßt weiterhin die Verwendung der erfindungsgemäßen Verbindungen bei der Herstellung von Arzneimitteln, die zur Behandlung und Prophylaxe der vorstehend genannten Krankheiten eingesetzt werden.

Ein weiterer Gegenstand der Erfindung sind Arzneimittel, die ein oder mehrere erfindungsgemäße Verbindungen der Formel I enthalten.

Die Arzneimittel werden nach an sich bekannten, dem Fachmann geläufigen Verfahren hergestellt. Als Arzneimittel werden die erfindungsgemäßen pharmakologisch wirksamen Verbindungen (= Wirkstoff) entweder als solche oder vorzugsweise in Kombination mit geeigneten pharmazeutischen Hilfs- oder Trägerstoffen in Form von Tabletten, Dragees, Kapseln, Suppositorien, Emulsionen, Suspensionen oder Lösungen eingesetzt, wobei der Wirkstoffgehalt bis etwa 95 %, vorteilhafterweise zwischen 10 und 75 % beträgt. Bei der topischen Anwendung reichen schon Wirkstoffkonzentrationen von 0,001 - 1 %, bevorzugt 0,01 -0,1 %, aus.

Geeignete Hilfs- bzw. Trägerstoffe für die gewünschte Arzneimittelformulierung sind beispielsweise neben Lösemitteln, Gelbildnern, Suppositoriengrundlagen, Tabletten-Hilfsstoffen und anderen Wirkstoffträgern auch Antioxidantien, Dispergiermittel, Emulgatoren, Entschäumer, Geschmackskorrigentien, Konservierungsmittel, Lösungsvermittler oder Farbstoffe.

Die Wirkstoffe können oral, parenteral (subkutan), topisch oder rectal appliziert werden, wobei die topische Applikation bevorzugt ist.

Die aktiven Verbindungen werden mit den dafür geeigneten Zusatzstoffen wie Trägerstoffen, Stabilisatoren oder inerten Verdünnungsmitteln vermischt und durch die üblichen Methoden in geeignete Darreichungsformen gebracht, wie Tabletten, Dragees, Steckkapseln, wäßrige alkoholische oder ölige Suspensionen oder wäßrige oder ölige Lösungen, Cremes oder Salben. Als inerte Trägerstoffe können z. B. Gummi arabicum, Magnesia, Magnesiumcarbonat, Kaliumphosphat, Milchzucker, Glukose oder Stärke, insbesondere Maisstärke, verwendet werden. Dabei kann die Zubereitung sowohl als Trocken- als auch als Feuchtgranulat erfolgen. Als ölige Trägerstoffe oder Lösemittel kommen beispielsweise pflanzliche oder tierische Öle in Betracht, wie Sonnenblumenöl oder Lebertran.

Zur subkutanen oder intravenösen Applikation werden die aktiven Verbindungen gewünschtenfalls mit den dafür geeigneten Substanzen wie Lösungsvermittler, Emulgatoren oder weiteren Hilfsstoffen in Lösung, Suspension oder Emulsion gebracht. Als Lösungsmittel kommen z. B. in Frage physiologische Kochsalzlösung oder Alkohole, z. B. Ethanol, Propanol, Glycerin, daneben auch Zuckerlösungen wie Glucose- oder Mannitlösungen, oder auch eine Mischung aus den verschiedenen genannten Lösungsmitteln.

Nachfolgend ist die Erfindung anhand von Beispielen näher erläutert.

## Beispiele

### Allgemeine Verfahrensvorschriften:

### Herstellung von Nigericinmesylat (Formel V, Z = $SO_2CH_3$)

725 mg (1 mmol) Nigericin werden mit 1,5 mmol $ClSO_2CH_3$ für 0,5 h in Pyridin (10 ml) gerührt. Nach der Zugabe von Wasser werden weitere 20 min bei Raumtemperatur gerührt. Die wäßrige Phase wird mit Ethylacetat (3 x 20 ml) extrahiert und die organische Phase wird mit 2 x 10 ml 0,1 N HCl und 2 x 10 ml Wasser gewaschen. Nach dem Trocknen über Natriumsulfat wird in Vakuum einrotiert. Chromatographie an 250 g Kieselgel mit Chloroform/Methanol von 30 : 1 auf 9 : 1 ergibt das Nigericinmesylat.

**Verfahren 1:**

1 mmol Nigericinmesylat (Formel V, Z = $SO_2CH_3$) werden in 30 ml Toluol gelöst und mit 5 mmol Tetrabutylammoniumverbindung für 3 - 40 h auf 60° C erhitzt. Die organische Phase wird 2 mal mit 10 ml Wasser gewaschen und anschließend im Vakuum einrotiert. Chromatographie an Kieselgel mit einem Gradienten von $CHCl_3$/MeOH von 40 : 1 auf 9 : 1 ergibt die reinen Verbindungen.

**Verfahren 2:**

1 mmol Nigericinmesylat werden in 20 ml DMF mit 10 mmol Natriumazid für 40 h auf 90° C erhitzt. Anschließend wird mit 100 ml Diethylether verdünnt und mit 4 mal 50 ml Wasser gewaschen. Nach dem Einrotieren der organischen Phase wird der Rückstand an Kieselgel chromatographiert. Die Elution erfolgt wie unter Verfahren 1 beschrieben.

**Verfahren 3:**

1 mmol Nigericinmesylat werden in 30 ml Alkohol gelöst und mit 1 mmol NaH für 6 - 18 h auf 60° C erhitzt. Nach dem Neutralisieren mit $NH_4Cl$ wird bis zu einem Sirup eingedampft und wie unter Verfahren 1 beschrieben chromatographiert.

**Verfahren 4**

1 mmol Nigericin werden in 30 ml Pyridin gelöst und mit 1,3 mmol t-Butyldiphenylsilylchlorid für 20 h bei Raumtemperatur gerührt. Nach der Hydrolyse mit Wasser (100 ml) wird mit Ethylacetat (3 mal 30 ml) extrahiert. Die organische Phase wird mit 50 ml Wasser und 50 ml 0,1 N HCl gewaschen. Nach dem Trocknen wird die Lösung einrotiert und wie unter Verfahren 1 beschrieben chromatographiert.

**Verfahren 5**

1 mmol Nigericinmesylat werden in 25 ml THF gelöst und mit 10 mmol Reagens und gegebenenfalls 0,3 mmol Triethylamin oder NaH für 6 - 30 h unter Rückfluß erhitzt. Nach der Zugabe von 30 ml Wasser wird mit 3 mal 30 ml Ethylacetat extrahiert. Die organische Phase wird mit 30 ml Wasser und 30 ml 0,1 N HCl gewaschen, getrocknet und einrotiert. Die Chromatographie an Kieselgel erfolgt wie unter Verfahren 1 beschrieben.

Die in der nachfolgenden Tabelle 1 aufgeführten Verbindungen wurden analog den oben beschriebenen Verfahren 1 bis 5 hergestellt. Angegeben sind: Die Ausgangsverbindung (Nigericinmesylat, Formel V: Z = $SO_2$-$CH_3$ oder Nigericin), das Lösungsmittel, die Reaktionstemperatur, das Reagens, die Ausbeute sowie das Analogieverfahren (ausgewählt aus den oben beschriebenen Verfahren 1 bis 5). Tabelle 2 gibt ausgewählte analytische Daten der erhaltenen Verbindungen an (C-, H-Analysen, Molmassenpeak der Natriumverbindung im Massenspektrum).

Tabelle 1:

| Beispiel | X | MG | Ausgangs-verbindung | Lösungs-mittel | Temperatur | Reagens | Ausbeute | Verfahren |
|---|---|---|---|---|---|---|---|---|
| 1 | F | 726,9 | V | Toluol | 60°C | $Bu_4NF$ | 60 % | 1 |
| 2 | Cl | 743,4 | V | Toluol | 60°C | $Bu_4NCl$ | 85 % | 1 |
| 3 | Br | 787,8 | V | Toluol | 60°C | $Bu_4NBr$ | 85 % | 1 |
| 4 | SCN | 766,0 | V | Toluol | 60°C | $Bu_4NSCN$ | 72 % | 1 |
| 5 | $NO_2$ | 754,02 | V | Toluol | 60°C | $Bu_4NNO_2$ | 78 % | 1 |
| 6 | $N_3$ | 750,1 | V | DMF | 90°C | $NaN_3$ | 42 % | 2 |
| 7 | $OCH_3$ | 738,99 | V | $CH_3OH$ | 60°C | $CH_3OH/NaH$ | 58 % | 3 |
| 8 | OBu | 781,1 | V | BuOH | 60°C | BuOH/NaH | 42 % | 3 |
| 9 | $-O(Ph)_2Si(t-Bu)$ | 936,34 | Nig | Pyridin | 20°C | $(t-Bu)(Ph)_2SiCl$ | 95 % | 4 |
| 10 | $SCH_2CH_3$ | 769,1 | V | THF | 60°C | $CH_3CH_2SH/Et_3N$ | — | 5 |
| 11 | 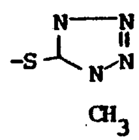 | 823,01 | V | THF | 60°C | 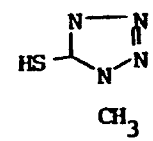 | — | 5 |
| 12 | Bz-N-Et | 842,21 | V | THF | 60°C | Bz-NH-Et | — | 5 |
| 13 | 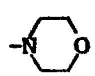 | 794,12 | V | THF | 60°C | 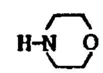 | — | 5 |

Bu = n-Butyl
t-Bu = tertiär-Butyl
Ph = Phenyl
Et = Ethyl

Bz = Benzyl
Nig = Nigericin
V = Nigericinmesylat (Formel V: X = $SO_2-CH_3$)

Tabelle 2

| Bsp. Nr. | C,H ber. | | C,H gef. | | FAB MS | Summenformel |
|---|---|---|---|---|---|---|
| 1 | C 66,1 | H 9,3 | C 66,0 | H 9,0 | 748 | $C_{40}H_{67}O_{10}F$ |
| 2 | C 64,6 | H 9,1 | C 64,8 | H 9,0 | 765 | $C_{40}H_{67}O_{10}Cl$ |
| 3 | C 61,0 | H 8,6 | C 61,4 | H 8,6 | 809 | $C_{40}H_{67}O_{10}Br$ |
| 4 | C 64,3 | H 8,8 | | | 788 | $C_{41}H_{67}O_{10}Sn$ |
| 5 | C 63,7 | H 8,8 | C 63,4 | H 8,8 | 776 | $C_{40}H_{67}O_{12}N$ |
| 6 | C 64,1 | H 9,0 | C 64,4 | H 9,4 | 772 | $C_{40}H_{67}O_{10}N_3$ |
| 7 | C 66,6 | H 9,5 | C 66,3 | H 10,1 | 761 | $C_{41}H_{70}O_{11}$ |
| 8 | | | | | 803 | $C_{44}H_{76}O_{11}$ |
| 9 | | | | | 958 | $C_{56}H_{86}O_{11}Si$ |
| 10 | | | | | 791 | $C_{42}H_{72}O_{10}S$ |
| 11 | | | | | 845 | $C_{42}H_{70}O_{10}N_4S$ |
| 12 | | | | | 864 | $C_{49}H_{79}O_{10}N$ |
| 13 | | | | | 816 | $C_{44}H_{75}O_{11}N$ |
| FAB MS = Molmassenpeak der Natriumverbindung im Massenspektrum<br>ber. = berechnet<br>gef. = gefunden | | | | | | |

**Antibakterielle Wirksamkeit**

Die antibakterielle Wirksamkeit der erfindungsgemäßen Verbindungen wurde im Agar-Verdünnungstest nach Lorian (Antibiotics in Laboratory Medicine, Williams & Wilkins, Baltimore/London, 1980) bestimmt. Dabei wurden die Präparate in einer geometrischen Verdünnungsreihe mit dem Faktor 2 in Müller Hinton Agar verdünnt. Eine Petrischale enthielt nur Müller Hinton Agar und diente zur Kontrolle des Bakterienwachstums. Die Petrischalen wurden dann mit einem Denley-Multipoint-Inokulator, welcher 0,6 µl einer 1 : 100 verdünnten 18-Stunden-Kultur der Testbakterien übertrug, mit den entsprechenden Testbakterien beimpft. Nach 16 bis 18 Stunden Inkubation bei 37 °C wurden die Petrischalen makroskopisch auf Bakterienwachstum untersucht.

Die niedrigste Konzentration der erfindungsgemäßen Verbindungen, welche das Bakterienwachstum noch vollständig verhinderte, wurde als MIC (Minimum Inhibitory Concentration) angenommen.

In Tabelle 3 ist die Wirkung (Angabe des MIC in µg/ml) von verschiedenen erfindungsgemäßen Verbindungen gegen folgende Bakterien aufgeführt:
Staph. aureus (SG 511), Staph. aureus (285),
Staph. aureus (503), Strept. pyogenes (308 A),
Strept. pyogenes (77 A), Strept. faecium (D).

Tabelle 3

| MIC in µg/ml | | | | | | |
|---|---|---|---|---|---|---|
| Verb. aus Bsp. | Staph. aureus (SG 511) | Staph. aureus (285) | Staph. aureus (503) | Strept. pyogenes (308 A) | Strept. pyogenes (77 A) | Strept. faecium (D) |
| 2 | 0,098 | 0,098 | 0,025 | 0,013 | 0,007 | 0,391 |
| 3 | 0,013 | 0,013 | 0,007 | < 0,002 | < 0,002 | 0,098 |
| 4 | 6,78 | 0,78 | 0,78 | 0,098 | 0,098 | 0,78 |
| 5 | 0,049 | 0,049 | 0,025 | 0,004 | 0,004 | 0,098 |
| 7 | 0,78 | 0,78 | 0,39 | 0,39 | 0,19 | 3,13 |
| 8 | 0,78 | 1,56 | 0,78 | 0,19 | 0,098 | 3,13 |
| 9 | 0,78 | 0,78 | 0,78 | 0,025 | 0,025 | 0,7 |

**Antivirale Wirksamkeit**

Antivirale Aktivität in der Zellkultur

Die Prüfsubstanzen wurden in Zellkulturmedium (Dulbecco's MEM) gelöst und in einer geometrischen Verdünnungsreihe, Faktor 3, in Standard-Mikrotiterplatten in 100 µl Zellkulturmedium vorgelegt. Anschließend erfolgte die Zugabe von 100 µl einer Suspension von HeLa- bzw. Vero-Zellen in Medium mit 5 % fötalem Kälberserum in einer Zelldichte von 2 x $10^5$ Zellen/ml. Die Ansätze wurden mit 50 µl einer Suspension des jeweiligen Test-Virus infiziert, die so eingestellt war, daß die Zellen innerhalb von 72 h einen cytopathogenen Effekt (CPE) zeigten. Die Auswertung erfolgte durch mikroskopische Begutachtung des Zellrasens und photometrische Messung der Neutralrotaufnahme (Farbtest nach Finter). Als MHK wurde die Konzentration des Präparats angenommen (µg/ml), bei der etwa 50 % der Zellen die Infektion überlebten.

In Tabelle 4 ist die Wirkung (Angabe des MHK in µg/ml) von verschiedenen erfindungsgemäßen Verbindungen gegen folgende Viren aufgeführt:
Adeno 5, Vaccina, Herpes I, Herpes II, Influenza A, Paramyxo. III, Rhinovirus II.

In Spalte 9 ist die DTM (Dosis Tolerata Maxima) in µg/ml angegeben.

Tabelle 4

| MHK in µg/ml | | | | | | | | |
|---|---|---|---|---|---|---|---|---|
| Verb. aus Bsp. | Adeno 5 | Vaccina | Herpes I | Herpes II | Influenza A | Paramyxo. III | Rhino. II | DTM [µg/ml] |
| 2 | --- | 0,49 | 0,05 | < 0,02 | 0,05 | --- | 0,49 | 1,48 |
| 3 | 0,01 | --- | 0,01 | 0,3 | 0,03 | --- | 0,3 | 0,89 |
| 7 | 0,16 | 0,49 | 0,05 | 0,05 | 0,05 | --- | 1,48 | 4,44 |
| 8 | 0,16 | 4,44 | 4,44 | 0,16 | 0,05 | 4,44 | 4,44 | 13,3 |
| 9 | 0,05 | 4,44 | 0,05 | 0,05 | 0,05 | 4,44 | 4,44 | 4,44 |

## Ansprüche

1. Nigericinderivate der Formel 1

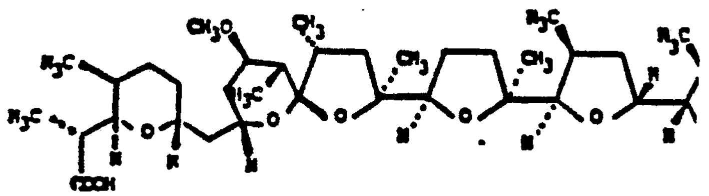

(I)

in der
X F, Cl, Br, J, SCN, CN, $NO_2$ oder $N_3$ bedeutet oder einen Substituenten der Formel II darstellt
Y - $R^1$ (II)
worin
$R^1$ Wasserstoff, $C_1$-$C_{10}$-Alkyl, $C_2$-$C_{10}$-Alkenyl, $C_3$-$C_8$-Cycloakyl, Trimethylsilyl, Triethylsilyl, Dimethylphenylsilyl, Dimethylcyclohexylsilyl, Dimethyltertiärbutylsilyl, Diphenyltertiärbutylsilyl, Pyrrolyl, Benzpyrrolyl, Imidazolyl, Benzimidazolyl, Triazolyl, Tetrazolyl oder Phenyl bedeutet, wobei die genannten Aryl-, Heteroaryl- oder benzokondensierten Heteroarylreste gegebenenfalls mit $C_1$-$C_4$-Alkyl, $C_1$-$C_4$-Alkoxy, $C_1$-$C_4$-Alkoxycarbonyl, Carboxyl, F, Cl, Br, J, $NO_2$ oder CN mono- oder disubstituiert sind und worin
Y O oder S bedeutet, wobei für
$OR^1$ die Bedeutungen Wasserstoff, Pyrrol, Benzpyrrol, Imidazol, Benzimidazol, Triazol und Tetrazol für $R^1$ ausgenommen sind und für
$SR^1$ die Bedeutungen Trimethylsilyl, Triethylsilyl, Dimethylphenylsilyl, Dimethylcyclohexylsilyl, Diphenyltertiärbutylsilyl und Dimethyltertiärbutylsilyl für $R^1$ ausgenommen sind oder worin
X einen Rest der Formel III

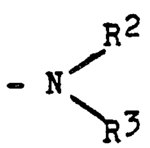

(III)

darstellt,
worin
$R^2$ und $R^3$ gleich oder verschieden sind und Wasserstoff, $C_1$-$C_{18}$-Alkyl, $C_2$-$C_{18}$-Alkenyl, $C_3$-$C_8$-Cycloalkyl oder Phenyl bedeuten, wobei die genannten Alkyl-, Alkenyl-und Cycloalkyl-Reste ihrerseits mit Phenyl, Naphthyl oder Thienyl monosubstituiert und die genannten Phenyl-und Alkylreste darüber hinaus noch mit $COOR^4$ monosubstituiert sein können, wobei $R^4$ Wasserstoff oder $C_1$-$C_6$-Alkyl bedeutet
oder worin
$R^2$ und $R^3$ zusammen mit dem N-Atom, an dem sie gebunden sind, einen 5- oder 6-gliedrigen N-haltigen Alkylring bilden, wobei eine $CH_2$-Einheit des Rings, welche nicht direkt benachbart zum N-Atom steht, ihrerseits durch NH, O oder S ersetzt sein kann,
sowie deren physiologisch verträgliche Salze.

2. Nigericinderivate der Formel I gemäß Anspruch 1, dadurch gekennzeichnet, daß mindestens eine der nachfolgenden Bedingungen erfüllt ist:
$R^1$ bedeutet Wasserstoff, $C_1$-$C_8$-Alkyl, $C_3$-$C_8$-Alkenyl, $C_3$-$C_8$-Cycloakyl, Trimethylsilyl, Triethylsilyl, Dimethyltertiärbutylsilyl, Dimethylphenylsilyl, Dimethylcyclohexylsilyl, Dimethyltertiärbutylsilyl, Pyrrolyl, Benzpyrrolyl, Tetrazolyl oder Phenyl, wobei die genannten Aryl-, Heteroaryl- oder benzokondensierten Heteroarylreste gegebenenfalls mit $C_1$-$C_4$-Alkyl, $C_1$-$C_4$-Alkoxy, $C_1$-$C_4$-Alkoxycarbonyl, Carboxyl, F, Cl, Br, J, $NO_2$ oder CN monosubstituiert sind;
$R^2$ und $R^3$ sind gleich oder verschieden und bedeuten Wasserstoff, $C_1$-$C_8$-Alkyl, $C_5$-$C_6$-Cycloalkyl oder Phenyl, wobei die genannten Alkyl- und Cycloalkylreste ihrerseits mit Phenyl monosubstituiert und die Phenyl-und Alkylreste darüber hinaus noch mit $COOR_4$ monosubstituiert sein können, wobei $R^4$ Wasserstoff oder $C_1$-$C_4$-Alkyl bedeutet.

3. Nigericinderivate der Formel I gemäß Anspruch 1 und/oder 2, dadurch gekennzeichnet, daß mindestens eine der nachfolgenden Bedingungen erfüllt ist:
X bedeutet F, Cl, Br, SCN, CN, $NO_2$ oder $N_3$ oder einen Substituenten der Formel II, worin
$R^1$ Wasserstoff, $C_1$-$C_6$-Alkyl, $C_5$-$C_6$-Cycloalkyl, Trimethylsilyl, Triethylsilyl, Diphenyltertiärbutylsilyl, Dimethylphenylsilyl, Dimethylcyclohexylsilyl, Dimethyltertiärbutylsilyl, Pyrrolyl, Benzpyrrolyl, Tetrazolyl, Methyltetrazolyl oder Phenyl bedeutet;
$R^2$ und $R^3$ sind gleich oder verschieden und bedeuten Wasserstoff, $C_1$-$C_4$-Alkyl oder Phenyl, wobei die Alkylreste ihrerseits mit Phenyl monosubstituiert sein können;
$R^2$ und $R^3$ bilden zusammen mit dem N-Atom, an dem sie gebunden sind, einen Morpholin-, Thiomorpholin- oder Piperazinring.

4. Verfahren zur Herstellung von Nigericinderivaten der Formel I, dadurch gekennzeichnet, daß man Nigericin (Formel I: X = OH) mit einer einen nukleofugen Rest liefernden Verbindung IV
Hal - Z (IV)
worin
Hal Chlor, Brom oder Jod und

Z $-SO_2CH_3$, $-SO_2CF_3$, 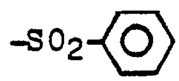 oder 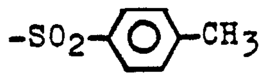

bedeuten,
zu einer Verbindung der Formel V

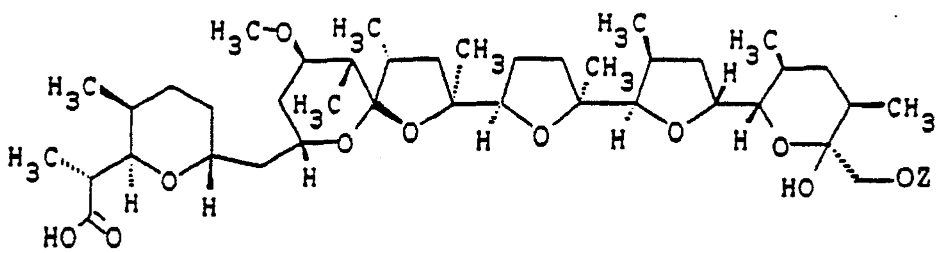

(V)

wobei Z die oben zu Formel IV angegebenen Bedeutungen hat, umsetzt,
und anschließend
a) die Verbindung V mit $NaN_3$ oder einer Verbindung der Formel VI umsetzt,
M - X′ (VI)
worin
M Li, Na, K oder ein quartäres, organisches Ammoniumfragment bedeutet und
X′ F, Cl, Br, J, SCN, CN oder $NO_2$ bedeutet, wobei man eine Verbindung der Formel I′ erhält,

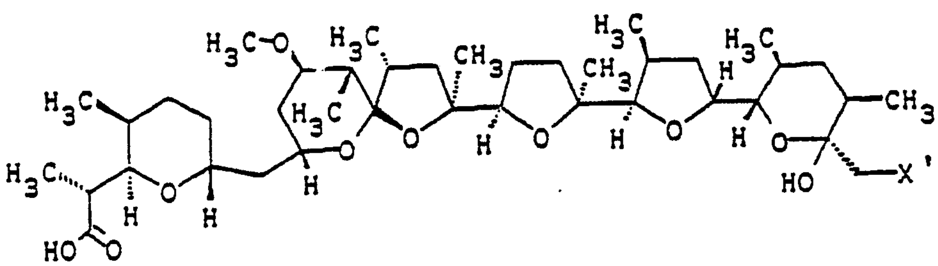

(I′)

worin
X′ $N_3$ ist oder die oben zu Formel VI angegebenen Bedeutungen hat
oder daß man
b) Nigericin oder die Verbindung der Formel V mit einem Alkohol, Thiol oder Amin der Formel VII bzw. VII′ umsetzt,

$HY - R^1$ (VII) 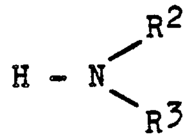 (VII')

worin
Y, $R^2$ und $R^3$ die oben zu Formel I angegebenen Bedeutungen haben und
$R^1$ bis auf Trimethylsilyl, Triethylsilyl, Dimethylphenylsilyl, Dimethylcyclohexysilyl, Diphenyltertiärbutylsilyl und Dimethyltertiärbutylsilyl
die oben zu Formel I angegebenen Bedeutungen hat, wobei man eine Verbindung der Formel I erhält, in der

X $Y-R^1$ oder 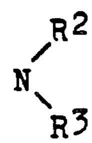

bedeutet, wobei Y, $R^2$ und $R^3$
die oben zu Formel I angegebenen Bedeutungen haben und $R^1$ bis auf Trimethylsilyl, Triethylsilyl, Dimethylphenylsilyl, Dimethylcyclohexylsilyl, Diphenyltertiärbutylsilyl und Dimethyltertiärbutylsilyl die oben zu Formel I angegebenen Bedeutungen hat
oder daß man
c) Nigericin umsetzt mit einer Verbindung der Formel VIII
Hal′ - Sil (VIII)
worin
Hal′ Chlor oder Brom und
Sil Trimethylsilyl, Triethylsilyl, Dimethylphenylsilyl, Dimethylcyclohexylsilyl, Diphenyltertiärbutylsilyl oder Dimethyltertiärbutylsilyl bedeutet,
wobei man eine Verbindung der Formel I erhält, in der
X $Y-R^1$ bedeutet, wobei Y Sauerstoff ist und
$R^1$ Trimethylsilyl, Triethylsilyl, Dimethylphenylsilyl, Dimethylcyclohexysilyl, Diphenyltertiärbutylsilyl oder Dimethyltertiärbutylsilyl bedeutet,
und daß man gegebenenfalls anschließend die Verbindungen der Formel I in ihre physiologisch verträglichen Salze überführt.

5. Arzneimittel, enthaltend eine Verbindung der Formel I gemäß Anspruch 1.

6. Verbindungen nach Anspruch 1 zur Verwendung als Arzneimittel.

7. Verbindungen nach Anspruch 1 zur Verwendung als antiviral und antibakteriell wirkende Arzneimittel.

8. Verfahren zur Herstellung von Arzneimitteln, welche antiviral und/oder antibakteriell wirken, dadurch gekennzeichnet, daß man in das Arzneimittel eine oder mehrere verschiedene Verbindungen der Formel I gemäß Anspruch 1 einverleibt.